# EUROPEAN PATENT APPLICATION

(11) **EP 4 473 972 A1**
(43) Date of publication of application: **11.12.2024**
(21) Application number: 23177739.2
(22) Date of filing: 06.06.2023
(51) Int. Cl.: A61K 31/5377, A61K 31/167, A61K 31/17, A61K 31/433, A61K 31/436, A61K 31/502, A61K 31/7048, A61K 31/7076, A61K 33/243, A61K 45/06, A61P 35/00

(54) **COMBINATIONS COMPRISING AN ATR INHIBITOR WITH SYNERGISTIC EFFECT AGAINST GLIOMAS**

(71) Applicant: Eberhard-Karls-Universität Tübingen, 72076 Tübingen (DE)
(72) Inventor: Tabatabai, Ghazaleh, 72076 Tübingen (DE); Walter, Bianca, 71083 Herrenberg (DE); Kuhlburger, Laurence, 72070 Tübingen (DE); Merk, Daniel Josef Wolfgang, 71134 Aidlingen (DE)
(74) Representative: Witte, Weller & Partner Patentanwälte mbB

(57) **Abstract**

The present invention relates to a new pharmaceutical composition for the treatment and prophylaxis of a glioma comprising a first active agent combined with at least a second active agent, wherein the combination of said first and said second active agent results in a synergistic effect. The present invention also relates to a first active agent combined with at least a second active agent for use in the treatment and prophylaxis of a glioma wherein the combination of said first and said second active agent results in a synergistic effect. Finally, the invention relates to a method of treatment and prophylaxis of glioma in a subject in need, comprising the administration to the subject of said pharmaceutical composition or the combination of said first and said second active agent. The first agent is ATR inhibitor and the second agent is chosen from: cytostatic agent, GSK-3 inhibitor, mTOR inhibitor and PARP inhibitor.

## Description

The present invention relates to a new pharmaceutical composition for the treatment and prophylaxis of a glioma comprising a first active agent combined with at least a second active agent, wherein the combination of said first and said second active agent results in a synergistic effect. The present invention also relates to a first active agent combined with at least a second active agent for use in the treatment and prophylaxis of a glioma wherein the combination of said first and said second active agent results in a synergistic effect. Finally, the invention relates to a method of treatment and prophylaxis of glioma in a subject in need, comprising the administration to the subject of said pharmaceutical composition or the combination of said first and said second active agent.

### FIELD OF THE INVENTION

The present invention relates to the field of drug development, more particular the field of active agents for the treatment and prophylaxis of a glioma.

### BACKGROUND OF THE INVENTION

The DNA damage response (DDR) is a complex network for maintaining the genetic integrity in cells. This might occur, for example, by inducing cell cycle arrest upon DNA damage detection and providing cells with time to repair DNA damage. Particularly the repair of three main lesions is relevant in this context: DNA double strand breaks (DSBs), DNA single strand breaks (SSB) and translesion synthesis. If the DNA damage is too severe, the DDR pathways can also steer cells towards cell death or senescence.

The DDR displays strong anti-cancer activity by halting the cell cycle upon DNA lesions and preventing mutations and cancer onset. In fact, DDR genes are frequently mutated in cancers and germline mutations in DDR genes lead to hereditary cancer predisposition. Furthermore, defects of DDR pathways in cancer may lead to genomic instability which in itself has been described as a hallmark of cancer. Genomic instability and the concomitant replicative stress and endogenous DNA damage might represent a targetable vulnerability in tumors with DDR alterations. For example, poly(ADP-ribose) polymerase (PARP) inhibitors in breast cancer gene 1 (BRCA1) or BRCA2 mutated ovarian cancers exploit this cancer intrinsic vulnerability and lead to synthetic lethal interactions with clinical relevance. The investigation of other DDR inhibitors, synthetic lethal combinations and their therapeutic potential have thus very high translational relevance in cancer research.

Glioblastoma are aggressive and incurable primary tumors of the central nervous system with a limited spectrum of registered therapies after maximum safe resection including radiation therapy, alkylating chemotherapy and tumor-treating fields. The median overall survival is still only in the range of 1.5 years, even in selected clinical trials populations. Acquired resistance to therapy is one of the key challenges of glioblastoma treatment. Resistance to radiation therapy has been linked to an upregulation of DDR pathway genes, specifically the upregulation of the ataxia telangiectasia mutated (ATM)/ataxia telangiectasia and Rad3 related (ATR) pathway. Due to their central role in detecting DSBs and SSBs, targeting the ATR and ATM pathway has become a research focus in many cancer entities; see Sundar et al. (2017), Targeting ATR in cancer medicine. Current Problems in Cancer 41: 302-315.

The clinical application of ATR inhibitors is in early clinical development and can be challenging due to adverse events like bone marrow suppression after continuous AZD6738 dosing Dillon et al. (2017), Abstract CT084: A Phase I dose-escalation study of ATR inhibitor monotherapy with AZD6738 in advanced solid tumors (PATRIOT Part A). Cancer Research 77: CT084-CT084.

Recently a sensitization of cells towards ATR inhibition (ATRi) in glioma overexpressing basic helix loop helix (bHLH) transcription factors was detected and anti-glioma effects of ATRi were detected in LN229 glioma cells *in vitro;* see Koch et al. (2020), Experimental glioma with high bHLH expression harbor increased replicative stress and are sensitive toward ATR inhibition. Neuro-Oncology Advances 2.

Other preclinical studies observed a synergistic interaction of ATRi with temozolomide in O6-methylguanine-DNA methyltransferase (MGMT)-deficient glioma cells (see Jackson et al., (2019), Temozolomide Sensitizes MGMT-Deficient Tumor Cells to ATR Inhibitors. Cancer Res. 79: 4331-4338) as well as with the oncolytic CAN-2409/Ganciclovir system in experimental glioma (see Koch et al. (2022), Perturbing DDR signaling enhances cytotoxic effects of local oncolytic virotherapy and modulates the immune environment in glioma. Molecular Therapy - Oncolytics 26: 275-288.

Furthermore, combination treatments have been tested in Phase I trials such as combination of ATRi with Carboplatin in advanced-stage solid tumors; see Yap et al. (2021), Ceralasertib (AZD6738), an Oral ATR Kinase Inhibitor, in Combination with Carboplatin in Patients with Advanced Solid Tumors: A Phase I Study. Clinical Cancer Research 27: 5213-5224.

However, previous approaches of monotherapies or even combination therapies with ATR inhibitors for the treatment of gliomas could not convince and did not yield satisfactory results in a clinical context.

Against this background, it is the object of the present invention to provide new and improved combination therapies for the treatment of gliomas based on ATR inhibitors. In particular, such combination therapies are to be provided that significantly improve the therapeutic success of monotherapies with ATR inhibitors.

### SUMMARY OF THE INVENTION

This object is met by the provision of a pharmaceutical composition for the treatment and/or prophylaxis of a glioma, said pharmaceutical composition comprising, in therapeutically/prophylactically effective amounts, a first active agent combined with at least a second active agent, wherein the combination of said first and said second active agent, when administered to a subject, results in a synergistic effect, wherein said first active agent is an ATR inhibitor and said second active agent is selected from the group consisting of: cytostatic agent, GSK-3 inhibitor, mTOR inhibitor, and PARP inhibitor.

The inventors used genome-wide CRISPR/Cas9 loss-of function and activation screens, thereby molecular targets were discovered which could potentiate ATR inhibition (ATRi) effects. They validated selected druggable targets by a drug library and subsequent functional assays *in vitro, ex vivo* and *in vivo.* The validated targets can be addressed by agents that, according to the inventors' findings, can be divided into four groups: cytostatic agent, GSK-3 inhibitor, mTOR inhibitor, and PARP inhibitor.

According to the invention "ATR" or 'ataxia telangiectasia and Rad3-related protein' or 'FRAP-related protein 1' (FRP1) is an enzyme that, in humans, is encoded by the ATR gene. ATR is a serine/threonine-specific protein kinase that is involved in sensing DNA damage and activating the DNA damage checkpoint, leading to cell cycle arrest in eukaryotes.

"ATR inhibitors" refer to a group of agents which can selectively and specifically target ATR and reduce or even block its enzymatic activity.

A "cytostatic agent" is a substance that slows or stops the growth of cells, including cancer cells. These agents may cause tumors to stop growing and spreading.

"GSK-3 or 'glycogen synthase kinase 3' is a serine/threonine protein kinase that mediates the addition of phosphate molecules onto serine and threonine amino acid residues. GSK-3 has been implicated in a number of diseases, including cancer.

"GSK-3 inhibitors" refer to a group of agents which can selectively and specifically target GSK-3 and reduce or even block its enzymatic activity.

"mTOR inhibitors" are a class of substances that inhibit the mechanistic target of rapamycin (mTOR), which is a serine/threonine-specific protein kinase that belongs to the family of phosphatidylinositol-3 kinase (PI3K) related kinases (PIKKs). mTOR regulates cellular metabolism, growth, and proliferation.

In conclusion, the inventors were able to demonstrate that the combination of the first and at least second active agents results in a synergistic effect with respect to the treatment or prophylaxis of a glioma. "Synergy" according to the invention means that the at least two active agents reinforce each other's effect such that the total effect of the active agents is stronger than a summation (potentiation or superadditive effect).

Methods to determine synergistic effects of at least two active agents are well known in the art, including the Bliss independence model and the Zero interaction potency (ZIP) model; see, e.g., Ma and Motsinger-Reif (2019), Current Methods for Quantifying Drug Synergism, Proteom Bioinform. 1(2): 43-48, and Yadav et al. (2015), Searching for Drug Synergy in Complex Dose-Response Landscapes Using an Interaction Potency Model, Comput. Struct. Biotechnol. J. 13: 504-513.

According to the invention, "prophylaxis" means any measure intended to prevent a disease or pathologic condition, such as a glioma, from developing or progressing to a pathogenic form.

According to the invention, "treatment" or therapy refers to all measures aimed at positively influencing or improving a disease or pathologic condition, such as a glioma.

According to the invention "therapeutically/prophylactically effective amount" or, synonymously, "effective dose", refer to the amount or dose of the first and/or at least second active agent, that is sufficient to achieve the desired clinical outcome or improvement.

A "subject" according to the invention refers to any living being, including a vertebrate, a mammal, and a human being.

According to the invention the first and/or second active agents also include the respective pharmaceutically acceptable salts thereof, the solvates thereof and the solvates of the salts thereof. Solvates for the purposes of the invention refer to those forms of the active agents, which in the solid or liquid state form a complex by coordination with solvent molecules. Hydrates are a specific form of solvates in which the coordination takes place with water.

In the pharmaceutical composition the first and second active agents can be, in an embodiment of the invention, the only active agents. "At least" a second active agent means that, in alternative embodiments, a third, fourth, fifth etc. active agent is comprised by the pharmaceutical composition according to the invention.

It goes without saying that the pharmaceutical compositing according to the invention may comprise a pharmaceutically acceptable excipient or carrier.

"Pharmaceutically acceptable excipients or carriers" are well known to the skilled person. They allow a proper formulation of the compound according to the invention and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cotton-seed oil, safflower oil, sesame oil, olive oil, corn oil and soybean oil; glycols such as propylene glycol; esters, such as ethyl oleate and ethyl laurate; agar; buffering agents, such as magnesium hydroxide and aluminum hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Suitable pharmaceutical acceptable carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

"Comprising" according to the invention generally means encompassing all the specifically mentioned features as well as optional, additional unspecified ones. However, according to embodiments of the invention, "comprising" also includes "consisting of" which means that only those features are included which are explicitly specified.

The problem underlying the invention is hereby completely solved.

According to an embodiment of the invention said cytostatic agent is selected from the group consisting of: cisplatin, hydroxyurea, fludarabine phosphate, vorinostat, and etoposide.

As the inventors were able to establish in their studies based on cell culture experiments and using primary cultures from patient material, it is especially these specific cytostatic agents in combination with an ATR inhibitor that lead to a synergistic effect. For cisplatin this synergistic effect could even be demonstrated in an animal experiment *in vivo.* These cytostatic agents are therefore particularly well suited as second agents.

"Cisplatin" (cis-diammine dichloridoplatin; DDP) (CAS number 15663-27-1) is a cytostatic drug for the inhibition of cell growth or cell division and contains a complex-bound platinum atom. The effect is based on an inhibition of DNA replication due to cross-linking of two adjacent guanine bases of a DNA strand.

"Hydroxyurea", also hydroxycarbamide (INN) (CAS number 127-07-1), is a cytostatic drug whose action is based on inhibition of the enzyme ribonucleotide reductase, which reduces ribose to deoxyribose.

"Fludarabine phosphate" or fludarabine-5-dihydrogen phosphate (CAS number 75607-67-9) is a fluorinated nucleotide analog of vidarabine and belongs to the group of purine analogs. Fludarabine is phoshorylated in the body's cells after intravenous administration to the active metabolite fludarabine-ATP. It is effective in this form. In this regard, it prevents DNA synthesis due to inhibition of ribonucleotide reductase and DNA polymerase. In addition, the fludarabine nucleotide is incorporated into DNA, leading to apoptosis of the cell. Fludarabine phosphate thus comprises cytotoxic properties.

"Vorinostat" (rINN) (CAS number 149647-78-9) also known as suberoylanilide hydroxamic acid (suberoyl+anilide+hydroxamic acid abbreviated as SAHA) is a member of a larger class of compounds that inhibit histone deacetylases (HDAC).

"Etoposide" is a glycoside of podophyllotoxin extracted from the root of the evergreen American mayapple (*Podophyllum peltatum*) (CAS number 33419-42-0). It inhibits the enzyme topoisomerase II and suppresses cytokinesis. For this purpose, it causes the cell to enter the stage of apoptosis and die. Furthermore, etoposide also leads to single and double strand breaks in DNA.

In another embodiment of the invention said GSK-3 inhibitor is tideglusib.

Tideglusib (NP-12, NP031112) is a potent and irreversible small molecule glycogen synthase kinase 3 (GSK-3) inhibitor 28CAS number 865854-05-3). It is particularly suitable according to the invention, as the inventors have found.

In yet another embodiment of the invention the mTOR inhibitor is everolismus.

Everolismus is the 40-O-(2-hydroxyethyl) derivative of sirolimus and works similarly to sirolimus as an inhibitor of mammalian target of rapamycin (mTOR) (CAS number 159351-69-6). According to the inventors, this is a particularly suitable mTOR inhibitor.

In still another embodiment, the PAPR inhibitor is olaparib.

Olaparib is a PARP inhibitor, inhibiting poly ADP ribose polymerase (PARP), an enzyme involved in DNA repair (CAS number 763113-22-0). In the experiments conducted by the inventors, olaparib showed good synergistic activity in combination with the ATR inhibitor and is therefore highly suitable as at least a second agent.

In yet another embodiment of the invention the ATR inhibitor is selected from the group consisting of: AZD6738 (ceralasertib; CAS number 1352226-88-0), VE-822 (berzosertib; CAS number 1232416-25-9), BAY-1895344 (elimusertib; CAS number 1876467-74-1), VE-821 (CAS number 1232410-49-9), RP-3500 (camonsertib; CAS number 2417489-10-0), ATR-IN-21 (CAS number 2905312-17-4), ATR-IN-20, ETP-46464 (CAS number 2713577-93-4), CGK733 (CAS number 905973-89-9), NU6027 (CAS number 220036-08-8), ATR-IN-14 (CAS number 2765785-88-2), ATR-IN-10 (CAS number 2713577-93-4), ATR-IN-19 (CAS number 2648989-61-9), ATR-IN-15 (CAS number 2756665-52-6), ATR-IN-17 (CAS number 2761194-15-2), antitumor agent-28 (CAS number 2097499-67-5), ATR-IN-13 (CAS number 2758113-84-5), ATR-IN-18 (CAS number 2766407-55-8).

The inventors have carried out most of their experiments - by way of example - with the ATR inhibitors AZD6738 and/or VE-822, but can conclude that other ATR inhibitors can also be used according to the invention in an advantageous manner, in particular those mentioned in this embodiment.

In an embodiment of the pharmaceutical composition of the invention the ATR inhibitor is AZD6738 (ceralasertib) and/or VE-822 (berzosertib) and the second active agent is cisplatin.

In this embodiment, the two active ingredients are used that were exemplarily employed in the experiments and led to a particularly good synergistic effect.

Each of the first and/or second active agent, in another embodiment, is present per dosage unit of the pharmaceutical composition at a concentration that, upon administration into a subject, results in a concentration in the glioma cells which is approx. ≤ 2000 µM, preferably about ≤ 1000 µM, further preferably ≤ 500 µM, further preferably ≤ 200 µM, further preferably ≤ 150 µM, further preferably ≤ 100 µM, further preferably ≤ 50 µM, further preferably ≤ 25 µM, further preferably ≤ 20 µM, further preferably ≤ 10 µM, further preferably ≤ 5 µM, further preferably ≤ 4 µM, further preferably ≤ 3 µM, further preferably ≤ 2.5 µM, further preferably ≤ 2 µM, and further preferably ≤ 1 µM.

In the exemplary experiments, the inventors used 1.5 µM AZD6738 for LN229 and 1.4 µM AZD6738 for LNZ308 cells. These two concentrations were then combined with two concentrations of cisplatin (1 and 2.5 µM), fludarabine phosphate (10 and 20 µM) and hydroxyurea (150 and 200 µM).

This measure takes into account the findings of the inventors that setting the right therapeutic window by using the specified concentrations ensures on the one hand that the anti-glioma activity of the active agents is sufficient and on the other hand any cytotoxic effects are minimized.

In an embodiment of the invention the glioma addressed is a glioblastoma.

Glioblastoma, previously known as glioblastoma multiforme (GBM), is the most aggressive and most common type of cancer that originates in the brain, and has very poor prognosis for survival. There is no known method of preventing this cancer. Treatment usually involves surgery, after which chemotherapy and radiation therapy are used. However, the results achieved are often unsatisfactory and usually short-lived. Therefore, the invention provides a remedy here for the first time and provides an improved therapy and prophylaxis option.

In another embodiment of the invention the synergistic effect is determinable by the Bliss independence model and the Zero interaction potency (ZIP) model.

This measure has the advantage that the models are used which were also used by the inventors to find the combinations of active agents. They are therefore particularly suitable for determining the synergy. Both methods are well known to the skilled person; see, *e.g.,* Ma and Motsinger-Reif (*loc. cit*.) and Yadav et al. (*loc. cit*.)*.*

Another subject-matter of the invention relates to a first active agent combined with at least a second active agent for use in the treatment and/or prophylaxis of a glioma, wherein the combination of said first and said second active agent, when administered to a subject, results in a synergistic effect, wherein said first active agent is an ATR inhibitor and said second active agent is selected from the group consisting of: cytostatic agent, GSK inhibitor, mTOR inhibitor, and PARP inhibitor.

The embodiments, properties, advantages, and features of the pharmaceutical composition according to the invention apply in a corresponding way to the combination for use according to the invention.

A still further subject-matter of the invention relates to a method of treatment and/or prophylaxis of glioma in a subject in need, comprising the administration to said subject of the pharmaceutical composition according to the invention or the first active agent combined with at least a second active agent for use according to the invention.

The embodiments, properties, advantages, and features of the pharmaceutical composition according to the invention apply in a corresponding way to the method according to the invention.

In an embodiment of the method according to the invention the first and the second active agent are administered simultaneously. In another embodiment of the method according to the invention the first and the second active agent are administered separately.

Due to this method of administration, their respective benefits can be selectively exploited and external circumstances of treatment can be accommodated. Simultaneous administration facilitates administration and promotes patient compliance or adherence to therapy. Separate administration can induce a priming effect via the agent administered first.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments and examples resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

### BRIEF DESCRIPTION OF THE FIGURES

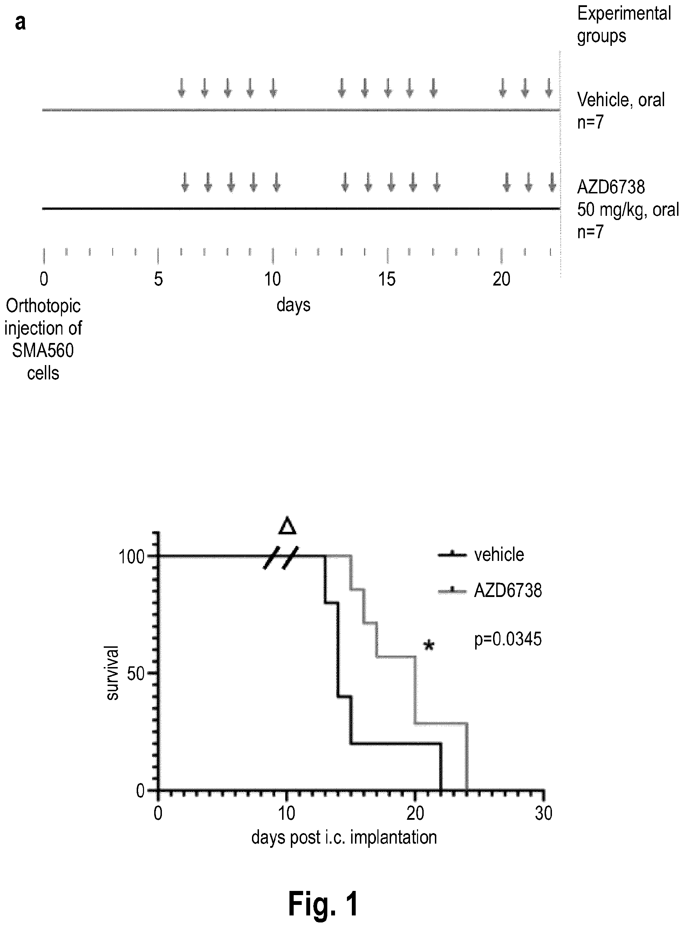

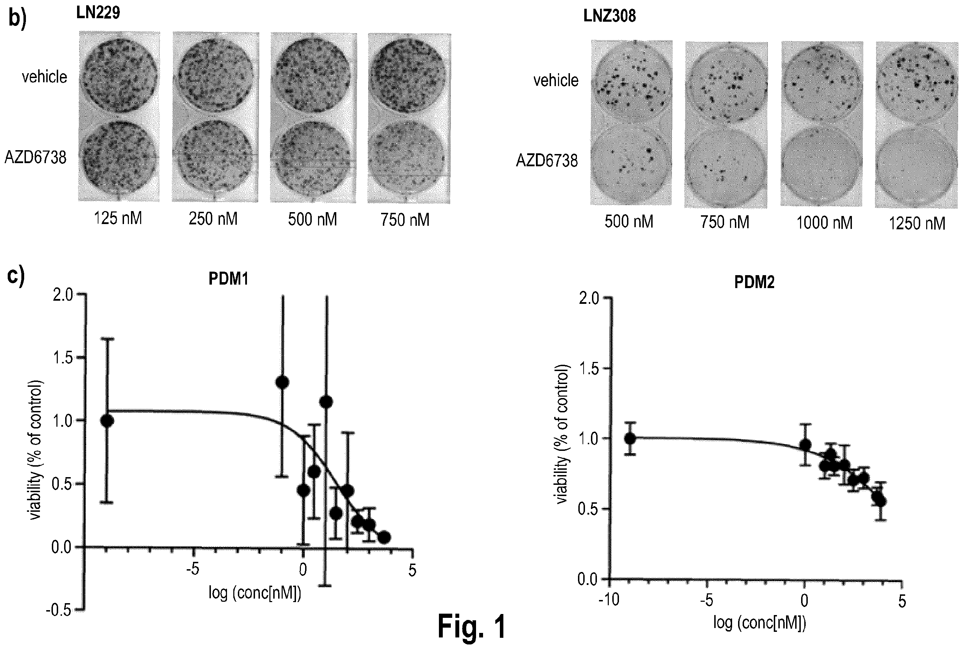

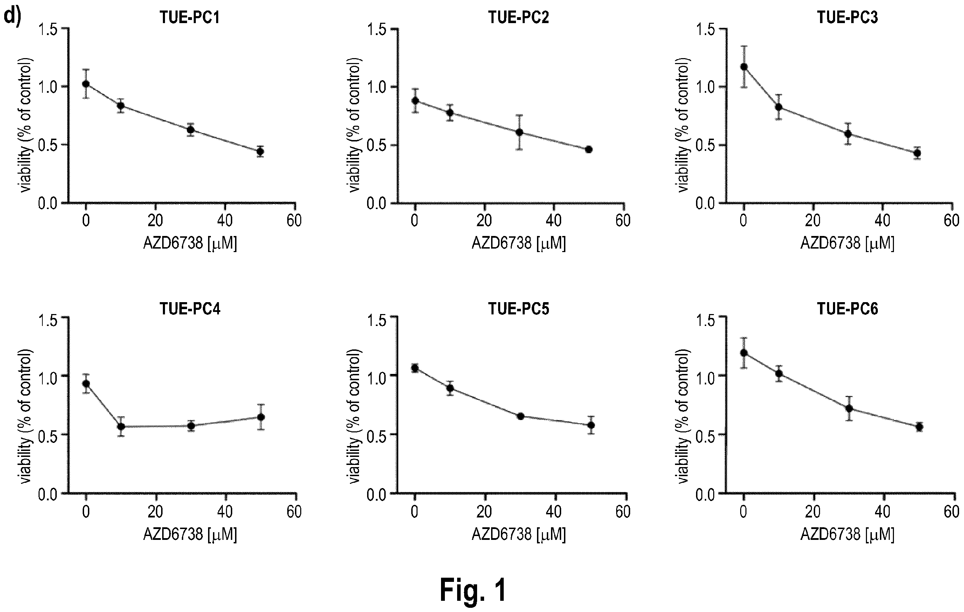

- Fig. 1: Anti-glioma activity of ATRi monotherapy in experimental glioma models. a, Left, treatment schedule, treatment started 6 days post-surgery (vehicle and AZD6738 groups: n=7). Right, Kaplan-Meier curve. Statistical analysis using Gehan-Breslow-Wilcoxon test, p-values < 0.05 considered significant. Δ Two animals in the control group were excluded from the analysis due to reasons unrelated to treatment or tumor development (eye infection). b, Clonogenic survival assay plates depicting a dose-dependent reduction of clonogenic survival in LN229 and LNZ308 cells treated with AZD6738. c, Dose-dependent reduction of viability of two patient-derived glioblastoma microtumor models (PDM) treated with AZD6738 for 72 h. Shown are means ± SD (n=3). d, Dose-dependent reduction of viability of six patient-derived primary cultures (TUE-PC) treated with AZD6738 for 72 h. Shown are means ± SD (n=1, 3 technical replicates per sample).

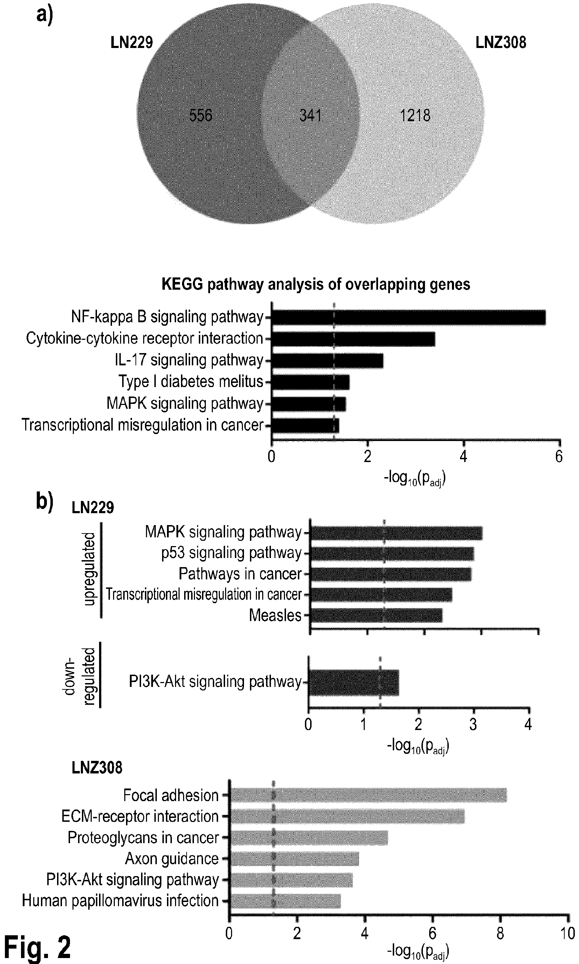

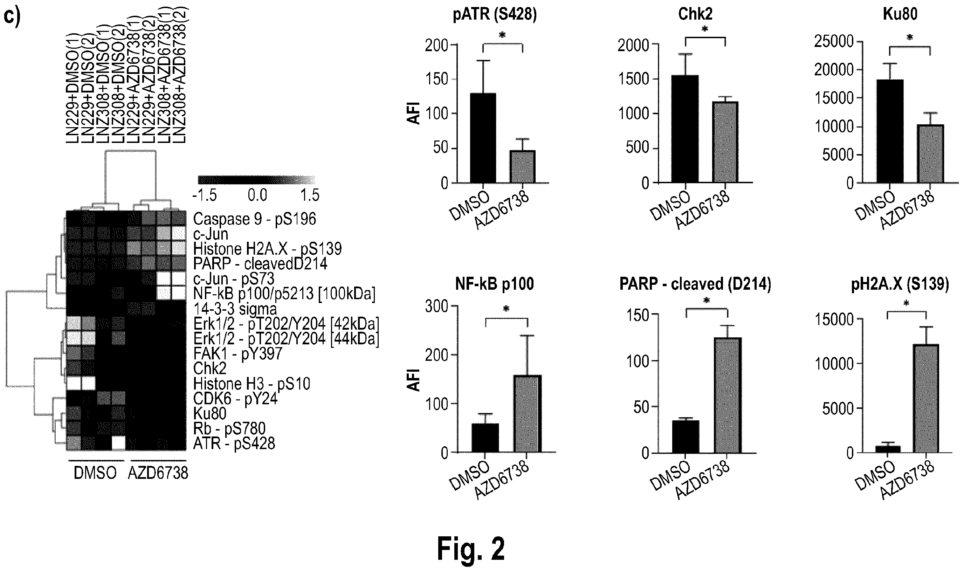

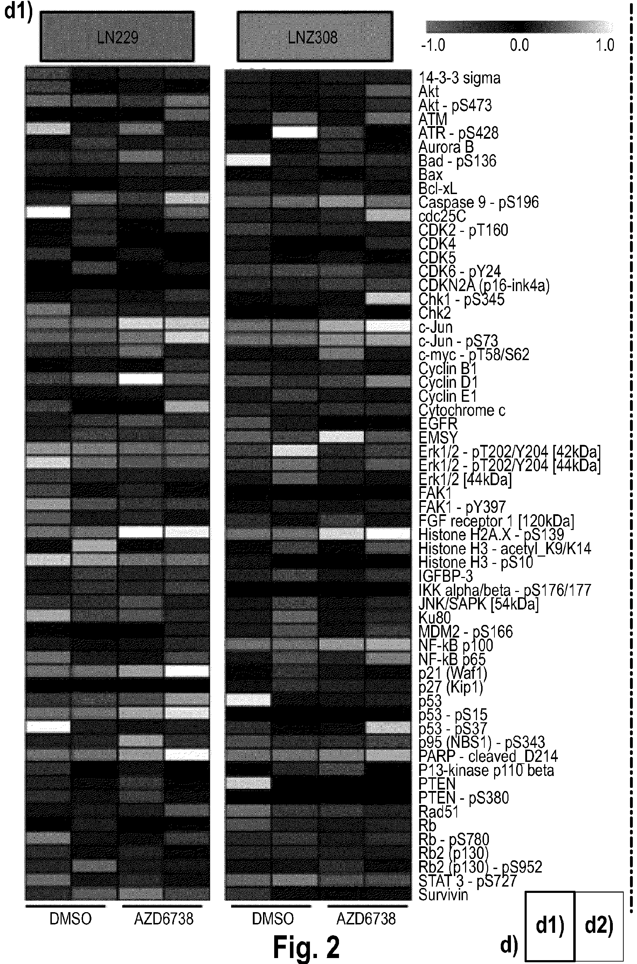

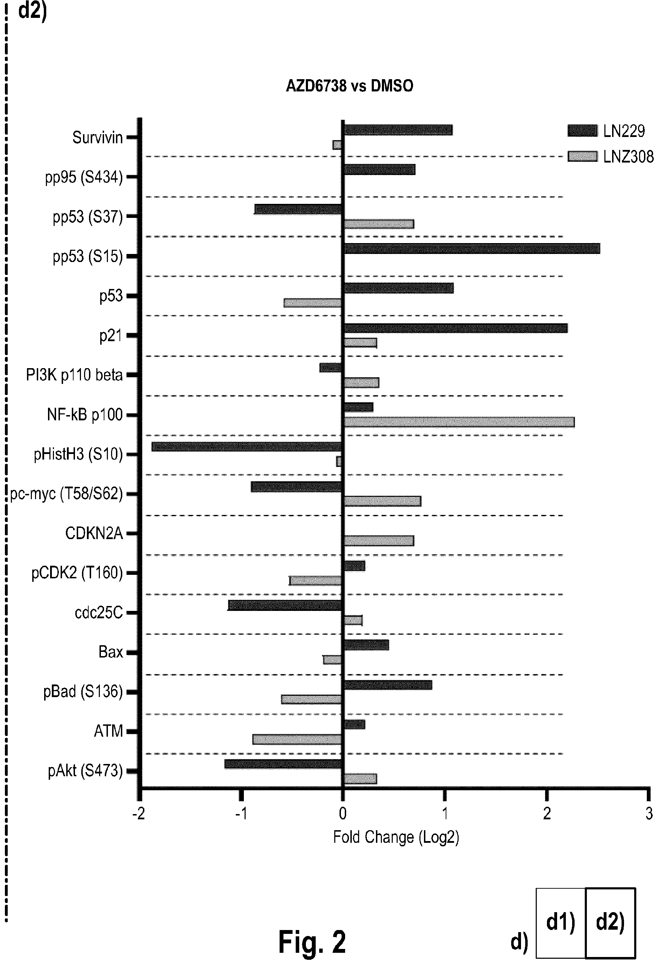

- Fig. 2: Transcriptomic and proteomic profiling before and after AZD6738 treatment, a, Venn diagram of upregulated differentially expressed genes (DEGs) in LN229 and LNZ308 cells treated with AZD6738 for 72 h. 341 upregulated genes are identified to overlap in both cell lines upon treatment. Lower panel depicts KEGG pathway analysis of identified overlapping genes. Red dashed line indicates significance level of p < 0.05. b, Based on the likelihood ratio test (LRT), genes identified to be differentially affected upon ATRi treatment in between cell lines are analyzed for KEGG pathway affiliation. p53 signaling is strongly upregulated in LN229 cells, PI3K-Akt signaling is downregulated in LN229 and upregulated in LNZ308 cells. Red dashed line indicates significance level of p < 0.05. c, DigiWest protein profiling heatmap depicting treatment- specific effects across both cell lines confirming target engagement (pATR), apoptosis induction (cleaved PARP), NPκB activation (NF-κB p100) and cell-cycle regulation (Chk2). Statistical analysis of significance for heatmap using Wilcoxon test (non-parametric, p<0.05), for bar graphs Mann-Whitney test (non-parametric, rank comparison, p<0.05. DMSO (n=4) vs. AZD6738 (n=4)). d, Left, heatmap depicting indicated analytes separated by cell line. Right, bar graph depicting analytes differentially regulated in both cell lines upon treatment. In line with transcriptomic data, p53 is upregulated in LN229 cells while pAkt is downregulated in LN229 cells and trends towards upregulation in LNZ308 cells upon treatment.

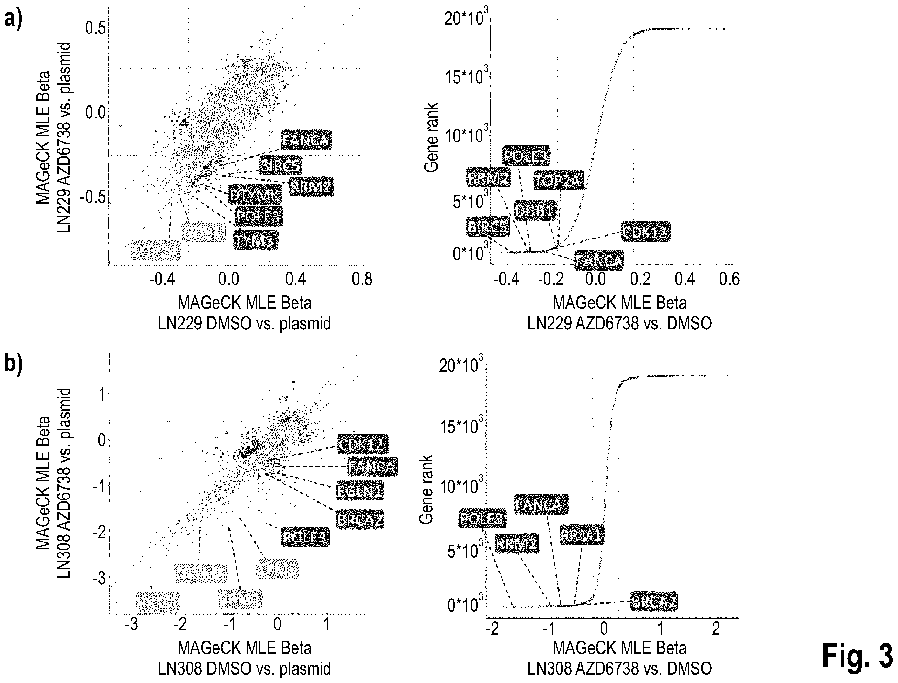

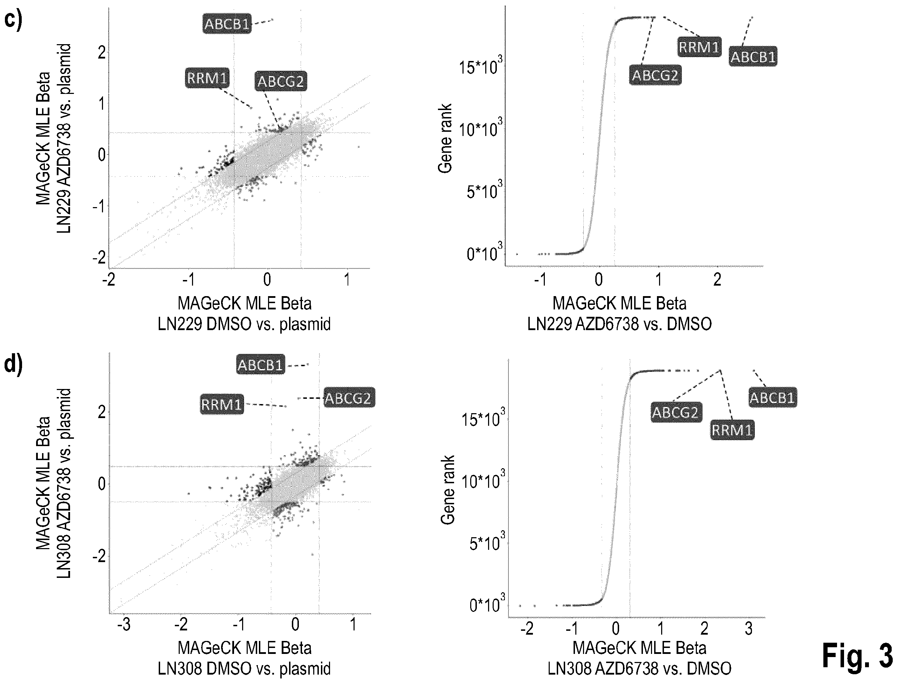

- Fig. 3: CRISPR-Cas9 genome-wide knockout and activation screens identify novel combination partners for AZD6738 treatment, a, b Upper half, CRISPR-Cas9 screen analyses using a knockout (Brunello) library in LN229 (a) and LNZ308 cells (b) using 750 nM of AZD6738. Left, 9-square plot of MAGeCK MLE results comparing Brunello sgRNA distributions from DMSO or AZD6738-treated cells to the plasmid library pool. Right, rankview plot illustrating MAGeCK MLE results comparing Brunello sgRNA distributions from AZD6738-treated cells to the corresponding DMSO control. c, d Lower half, CRISPR-Cas9 screen analyses using an activation (Calabrese) library in LN229 (c) or LNZ308 cells (d), respectively, using 1.5 µM AZD6738 for LN229 and 1.4 µM AZD6738 for LNZ308 cells. Left, 9-square plot of MAGeCK MLE results comparing Calabrese sgRNA distributions from DMSO or AZD6738-treated cells to the plasmid library pool. Right, rankview plot illustrating MAGeCK MLE results comparing Calabrese sgRNA distributions from AZD6738-treated cells to the corresponding DMSO control. The respective experimental set-up was used to prioritize genetic vulnerabilities (Brunello library) and resistance mechanisms (Calabrese library) upon ATR inhibition.

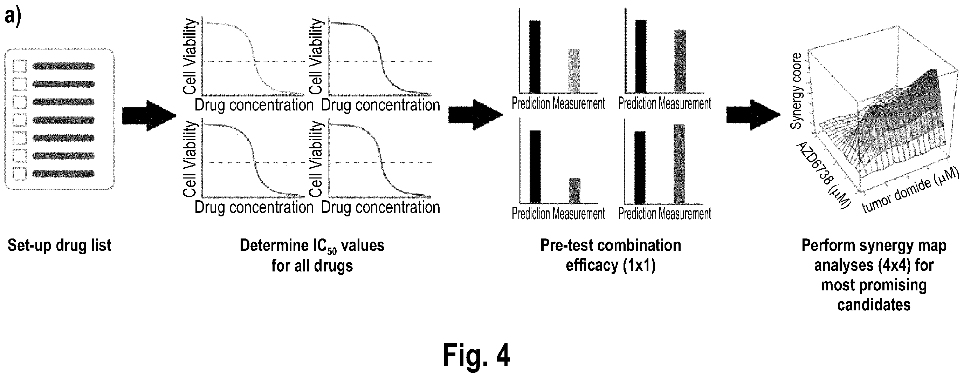

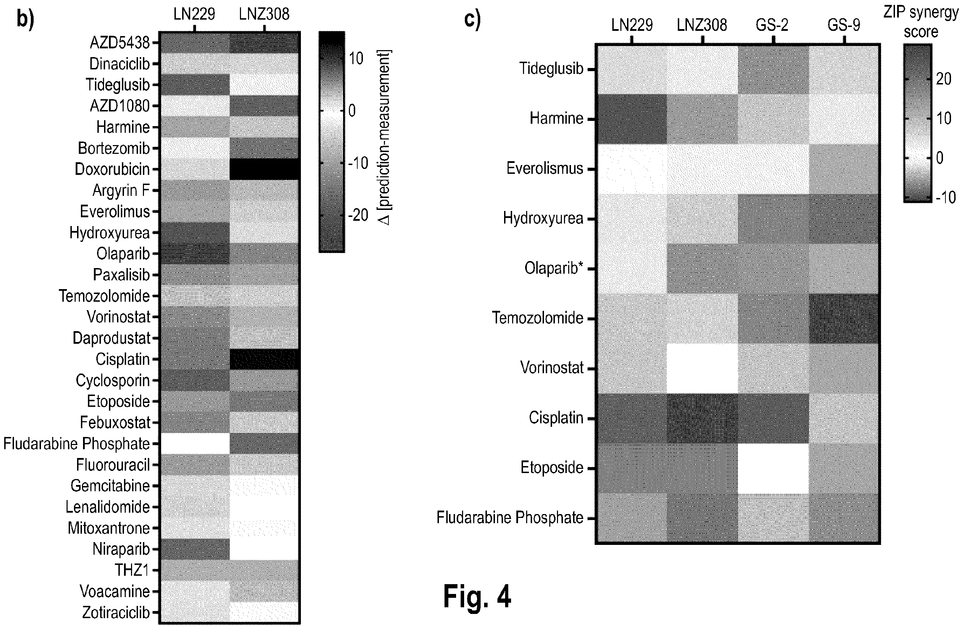

- Fig. 4: Functionally-instructed combination therapies *in vitro,* a, Schematic workflow of the functionally-instructed drug screen. For all selected drugs IC50 values were determined. Then, a pre-selection screen, i.e. 1x1 (IC50xIC50) combination of AZD6738 plus drug of interest, was set-up. All combinations with AZD6738 resulting in higher efficacy than additive interaction were then included in 4x4 synergy map analyses. b, Analysis of the 1x1 pre-selection screen (n=1 with 8 technical replicates per sample). The heatmap depicts the delta value between prediction of additive drug-drug interaction and measured viability (Bliss synergy score). Positive values (blue) indicate a higher efficacy of the drug combination than predicted, negative values (red) indicate a lower efficacy of the drug combi-nation than predicted, tideglusib, harmine, everolimus, hydroxyurea, olaparib, temozolomide, vorinostat, cisplatin, etoposide and fludarabine phosphate were selected as top candidates, c, Analysis of the 4x4 synergy map experiments. Heatmap depicts the average ZIP synergy score across tested combinations (n=2). Green coloring indicates high synergism scores, brown coloring negative synergism scores. Hydroxyurea, cisplatin and fludarabine phosphate show positive synergism values across all four cell lines tested. *In LN229 AZD6738/Olaparib analyses the highest AZD6738 concentration was removed from the calculation of median synergy scores.

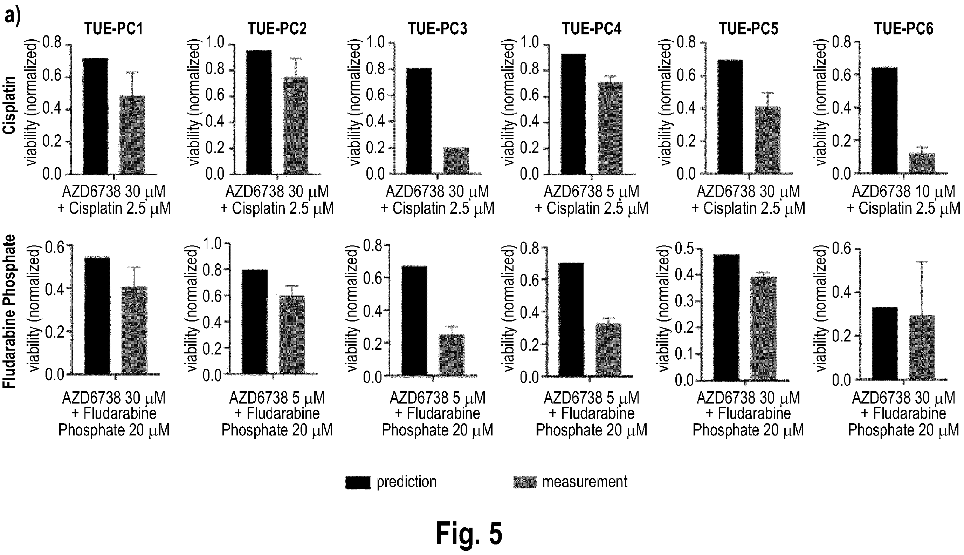

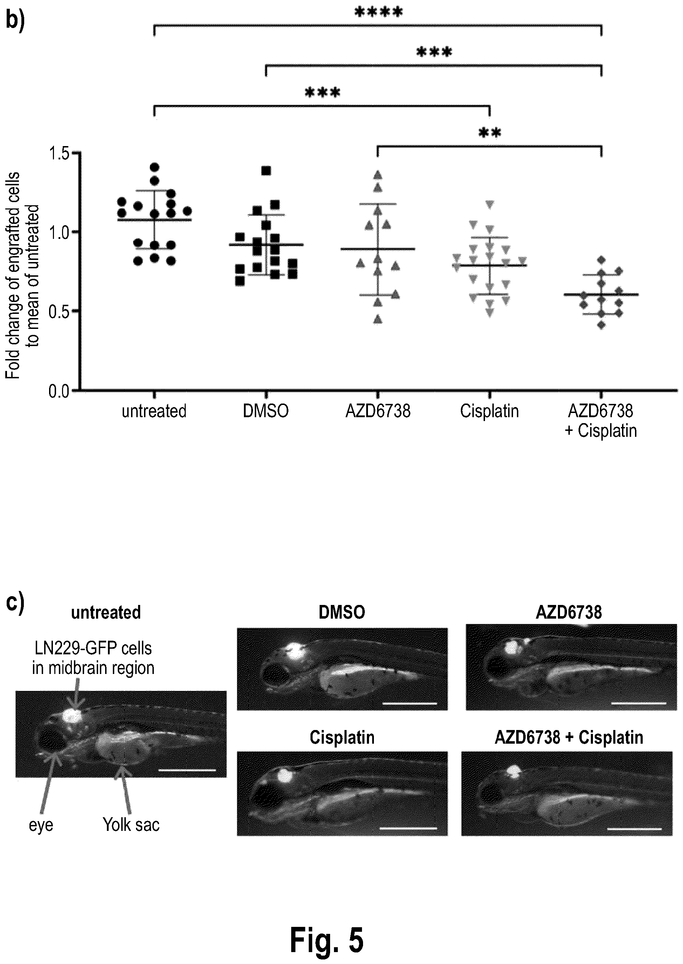

- Fig. 5: Combination of AZD6738 with cisplatin or fludarabine phosphate show synergistic efficacy *ex vivo* and *in vivo.* a, Evaluation of combination treatment efficacy and synergy of AZD6738 with cisplatin (upper panel) or with fludarabine phosphate (lower panel) in primary glioma cultures (PC). Shown as black bar is the predicted value for additive combination effects based on the Bliss Independence Criterion as outlined in Methods. Observed measurements (purple) of combination treatments are depicted in purple. Lower values than predicted indicate a synergistic effect of the combination. Shown are means ± SD (n=1, 3 technical replicates per sample). b, Tumor areas of control (untreated (n=16), DMSO (n=16)), AZD6738 (n=12), cisplatin (n=19) and the combination of both (n=12) treated wildtype zebrafish embryos xenotransplanted with LN229-GFP cells. Data was collected in two independent experiments. Tumor surface areas are measured using Imaris (version 9.2.0) after 48 h of treatment. Measurements were normalized to untreated control, means ± SD of the respective groups are indicated, each dot represents one embryo. Statistical analysis using one-way ANOVA (all to all comparison of means), Sidak correction for multiple testing, shown are only comparisons with corrected p-values < 0.5. * p<0.05, ** p<0.01, *** p<0.001, **** p<0.0001. Below graph, exemplary pictures of zebrafish embryos of each group. Left, untreated zebrafish embryo with anatomical features "eye", "yolk sac" and "LN229-GFP cells in midbrain region" highlighted by red arrows. Scale bars: 500 µm.

### EXAMPLES

### 1. General

The DNA damage response (DDR) is a physiological network preventing malignant transformation, e.g., by halting cell cycle progression upon DNA damage detection and promoting DNA repair. Glioblastoma are incurable primary tumors of the nervous system and DDR dysregulation contributes to acquired treatment resistance. Therefore, DDR targeting is a promising therapeutic strategy. The inventors investigate Ataxia telangiectasia and Rad3 related (ATR) inhibition (ATRi) and functionally-instructed combination therapies involving ATRi in experimental glioma. They were able to show that ATRi monotherapy prolongs survival *in vivo* and modulates the molecular network. Using genome-wide CRISPR/Cas9 loss-of function and activation screens, we discover molecular targets that could potentially further enhance ATRi effects. The inventors validated selected druggable targets by a drug library and subsequent functional assays *in vitro, ex vivo* and *in vivo.* In conclusion, the study leads to the identification of novel combination therapies involving ATRi that could inform future early phase clinical trials and preclinical studies.

### 2. Material and methods

### Syngeneic glioma mouse model

All animal experiments were approved by the regional council Tübingen and conducted in accordance with animal law. Animals used in this study are kept and bred in the animal facility of the institute. They are regularly analyzed for infectious diseases. The inventors injected 5000 SMA560 glioma cells into the right striatum of VM/Dk mice (male and female). On day 5 post surgery, they randomized mice into treatment and control arm. The inventors administered AZD6738 (celasertib, Selleckchem, Houston, TX, US) at 50 mg/kg bodyweight or vehicle control via oral gavage for five days followed by two days of treatment holiday. Animals were closely monitored as shown in the scoring sheets. The endpoint of the experiment was according to the animal law "the time until the onset of neurological symptoms", in the text designated as "survival".

### Long term cell lines and primary glioblastoma cell cultures

The inventors cultured LN229, LNZ308, GL-261 and SMA560 glioma cell lines in DMEM (Gibco, Carlsbad, CA, US) supplemented with 10% fetal calf serum (Thermo Fisher Scientific, Waltham, MA, US) and 50 µg/mL gentamycin (Thermo Fisher Scientific, Waltham, MA, US). GS-2 and GS-9 were cultured in Neurobasal^{®}-A Medium (Gibco, Carlsbad, CA, US) supplemented with 2% B27 without vitamin A (Invitrogen, Waltham, MA, US), 1% GlutaMAX (Thermo Fisher Scientific, Waltham, MA, US), 0.02 µg/mL fibroblast growth factor (FGF) and epidermal growth factor (EGF) (PeproTech, Cranbury, NJ, US) and 50 µg/mL gentamycin (Thermo Fisher Scientific, Waltham, MA, US). Standard culture conditions are 5% CO₂ at 37°C.

Primary tumor tissue was obtained from fresh residual material upon resection at the Department of Neurosurgery, University Hospital Tübingen. The inventors cut the tissue in small pieces, washed it using Hanks Balanced Salt Solution (HBSS) (Gibco, Carlsbad, CA, US) and digested it by collagenase and dispase (Roche, Basel, CH). To remove any remaining erythrocytes, they used red blood cell lysis buffer (Sigma Aldrich, St. Louis, MO, US). The inventors then cultured the cells as GS cells in Neurobasal^{®}-A medium. They first evaluated AZD6738 sensitivity of each primary culture and selected two concentrations. These two concentrations were then combined with two concentrations of cisplatin (1 and 2.5 µM), fludarabine phosphate (10 and 20 µM) and hydroxyurea (150 and 200 µM). To evaluate combination treatment analyses, the inventors leveraged the Bliss independence Criterion.

### Patient derived microtumors (PDM)

PDMs were extracted from surgically resected, residual glioblastoma tissue as is known to the skilled person. The inventors treated the PDMs with indicated drug concentrations for 72 h and determined cell viability using RealTime-Glo MT Cell Viability Assay (Promega, Madison, Wl, US). Luminescence assay signal was measured using a multimode microplate reader (Tecan, Männedorf, Switzerland). Obtained luminescence units were background corrected and plotted as dose response curves using GraphPad Prism 9 software. The use of residual tissue after tumor resections was approved by the ethical board of the University Hospital Tübingen.

### Functionally-instructed compound library

The inventors used AZD6738 (celasertib, Selleckchem, Houston, TX, US) at a stock concentration of 50 mM in dimethylsulfoxide (DMSO) and berzosertib (ChemiTek, Indianapolis, IN, US) also diluted at 50 mM in DMSO as ATR inhibitors. Furthermore, they generated a compound library based on the functionally-instructed targets for drug screens.

### Acute cytotoxicity assay

The inventors seeded 5,000 or 10,000 cells, respectively, on day one, treated the next day in serum-free medium and incubated for up to 72 h. Subsequently, they measured cell viability using CellTiterBlue reagent (Promega, Madison, WI, US) according to manufacturer's instructions with a GloMax (Promega, Madison, Wl, US). The inventors normalized measurements to untreated cells.

### Clonogenic survival assay

Cells were seeded the day before treatment. The inventors treated the cells in the indicated concentrations in serum-free medium for 24 h after which they changed the medium back to medium containing FCS, followed by a 7 to 21 day incubation time. The inventors used crystal violet (0.5% w/v) to stain colonies and analyzed the area coverage using the ImageJ software Plugln ColonyArea. Measurements were normalized to respective vehicle control wells.

### Annexin V/PI - flow cytometry analysis

For this, the FITC Annexin V Apoptosis Detection Kit I (Beckton, Dickinson & Company, Franklin Lakes, NJ, US) was used according to manufacturer's protocol. In brief, the inventors seeded the cells on day one and treated them on the next day. 48 h after treatment, they detached the cells, stained and analyzed them within 1 h on a MACSQuant Analyzer 10 (Miltenyi Biotec, Bergisch Gladbach, Germany). Next, the inventors analyzed the acquired data with the FlowJo software (Beckton, Dickinson & Company, Franklin Lakes, NJ, US).

### Cell cycle analysis

The inventors seeded the cells on day one and treated them on the next day. After 72 h, they detached the cells and stained them with propidium iodide solution (50 µg/mL propidium iodide (Thermo Fisher Scientific, Waltham, MA, US), 0.2% Triton X-100 (Roth, Karlsruhe, Germany), 100 µg RNase (Thermo Fisher Scientific, Waltham, MA, US), 1 g/L glucose in PBS (phosphate buffered saline) (Gibco, Carlsbad, CA, US). Flow cytometry was done using a MACSQuant Analyzer 10 (Miltenyi Biotec, Bergisch Gladbach, Germany). The inventors analyzed the acquired data with the FlowJo software (Beckton, Dickinson & Company, Franklin Lakes, NJ, US).

### RNA-Sequencing (RNAseq)

For this, the inventors seeded the cells on day one and treated them on the next day with IC50 concentrations of AZD6738 for up to 72 h. They extracted the RNA using the Qiagen RNeasy Mini kit (Qiagen, Venlo, NL) according to manufacturer's protocol. For RNA sequencing, the inventors enriched the mRNA fraction using polyA capture from 200 ng of total RNA using the NEBNext Poly(A) mRNA Magnetic Isolation Module (NEB). They used the NEB Next Ultra II Directional RNA Library Prep Kit for Illumina (NEB) to prepare mRNA libraries according to manufacturer's instructions. The libraries were sequenced as paired-end 50 bp reads on an Illumina NovaSeq6000 (Illumina) with a sequencing depth of approximately 25 million clusters per sample. The inventors performed RNA raw data QC and processing using megSAP (version 0.2-135-gd002274) combined with ngs-bits package (version 2019_11-42-gflb98e63). To align the reads to the GRCh38 they used STAR v2.7.3a and alignment quality was analyzed using ngs-bits. Normalized read counts for all genes were obtained using Subread (v2.0.0) and edgeR (v3.26.6). The inventors used the R package "DESeq2" for further analyses. They defined differentially expressed genes (DEGs) as log2 fold-change (LFC) above or below |1| and p-adjusted < 0.01 as defined by Wald statistics. The inventors analyzed the resulting hits for KEGG pathway association using gProfiler. They also used the likelihood ratio test (LRT) to identify significantly differentially changed genes between treated cell lines. They re-aligned significant LRT genes with DEG results and analyzed them with gProfiler.

### DigiWest Multiplex Protein Analysis

After 72 h of AZD6738 treatment, the inventors detached and counted the cells and subsequently snap froze them in liquid nitrogen. For protein preparation, they kept the cells on ice and added 20-30 µL of lysis buffer (LDS Lysis Buffer (Life Technologies, Carlsbad, CA, USA), supplemented with reducing agent (Thermo Fisher Scientific, Waltham, MA, USA) and protease- (Roche Diagnostics GmbH, Mannheim, Germany) and phosphatase-inhibitor (Roche)). Proteins were denatured by heating to 95°C for 10 min before the lysates were transferred to QiaShredder Eppendorf tubes (Eppendorf, Hamburg, Germany). After centrifugation (16,000g, 5 min, RT), eluates were stored at -80°C until further use. Protein quantification was performed using in-gel staining. 1 µL of each original lysate was diluted 1:10 (v/v) in lysis buffer. The respective aliquots were denatured for 10 mins at 70°C and 10 µL were run in a NuPAGE 4-12% Bis-Tris precast gel (Thermo Fisher Scientific) according to the manufacturer's instructions. The gel was washed with water and proteins were stained with BlueBandit (VWR, Darmstadt, Germany) for 1 h. The gel was de-stained over night with ddH2O before detection on a LI-COR (LI-COR, Bad Homburg, Germany) instrument. Analysis and protein quantification was performed using ImageStudio.

The DigiWest analysis was performed as described in the art. In brief, the inventors loaded 10 µg of cellular protein on an SDS-polyacrylamide gel and size-separated them using the commercial NuPAGE system (Life Technologies). Size-separated proteins were blotted onto a PVDF membrane and biotinylated on the membrane using NHS-PEG12-Biotin (50 µM) in PBST for 1 h. After drying of the membrane, the samples lanes were cut into 96 strips of 0.5 mm width using an automated cutting plotter (Silhouette America, West Orem, UT, USA) each corresponding to a defined molecular weight fraction. Each of the strips was placed in one well of a 96-well plate and 10 µl elution buffer (8 M urea, 1% Triton-X100 in 100 mM Tris-HCl pH 9.5) was added. The eluted proteins were diluted with 90 µl of dilution buffer (5% BSA in PBS, 0.02% sodium azide, 0.05% Tween-20) and each of the protein fractions was incubated with 1 distinct magnetic color-coded bead population (Luminex, Austin, USA) coated with neutravidin. The biotinylated proteins bind to the neutravidin beads such that each bead color represents proteins of one specific molecular weight fraction. All 96 protein loaded bead populations were mixed resulting in reconstitution of the original lane. Such a beadmix was sufficient for about 150 individual antibody incubations. Aliquots of the DigiWest bead-mixes (about 1/200th per well) were added to 96 well plates containing 50 µl assay buffer (Blocking Reagent for ELISA (Roche, Rotkreuz, Switzerland) supplemented with 0.2% milk powder, 0.05% Tween-20 and 0.02% sodium azide) and different diluted antibodies were added to the wells. After overnight incubation at 15°C in a shaker, the bead-mixes were washed twice with PBST and species-specific PE-labelled (Phycoerythrin) secondary antibodies (Dianova, Hamburg, Germany) were added and incubated for 1 hour at 23°C. Beads were washed twice prior to readout on a Luminex FlexMAP 3D.

For quantification of the antibody specific signals, we used the DigiWest Analyzer software; it automatically identifies peaks of appropriate molecular weight and calculates the peak area. Signal intensity was normalized to the total amount of protein loaded onto one lane. The software package MEV 4.9.0 was used for statistical analysis along with Graph Pad Prism (Version 9.0.0). For all statistical tests, a p-value <0.05 was considered significant.

### CRISPR/Cas9 genome-wide knockout screens

The genome-wide CRISPR/Cas9 knockout library Brunello was previously described and targets 19,114 human genes with 76,441 sgRNAs; see Doench et al. (2016), Optimized sgRNA design to maximize activity and minimize off-target effects of CRISPR-Cas9. Nature Biotechnology 34: 184-191. For generation of Cas9 expressing cells, glioma cell lines were transduced with a lentivirus coding for Cas9 (Addgene plasmid #52962; RRID:Addgene_52962) in the presence of polybrene (4 or 8 µg ml-1, respectively). Following transduction, the inventors transduced cells and selected them with a predetermined concentration of blasticidin for five days, and verified Cas9 expression using immuno blots.

For genome-wide knockout screens, the inventors transduced a total of 225*10^6 (LN229) and 200*10^6 (LNZ308) Cas9 expressing cells with a predetermined appropriate volume of lentiviral-packaged Brunello library to achieve a maximum 30% transduction efficiency maintaining a 500x library coverage. The inventors performed transductions in technical duplicates. 24 h after transduction, cells were selected with a predetermined concentration of puromycin for a total of five days, and transduction efficacy was evaluated using an in-line assay. Starting on day 7 of the screen, for each duplicate the inventors split 40*10^6 cells to drug or corresponding DMSO arms, thereby maintaining a coverage of 500x. They regularly split all screen arms under continuous drug or vehicle treatment for a total of 14 days, maintaining a minimum library coverage of 500x at all time. The inventors collected 60*10^6 cells, pelleted and stored them at -80°C for DNA extraction.

### CRISPR Cas9 genome-wide activation screens

Similarly to knockout screens outlined above, the inventors used CRISPR screens leveraging the previously described Calabrese P65-HSF library (Set A) for genome-wide gene activation; see Sanson et al. (2018); Optimized libraries for CRISPR-Cas9 genetic screens with multiple modalities. Nature Communications 9: 5416. This library targets a total of 18,885 gene promoters with a set of 56,762 sgRNAs. Glioma cell lines LN229 and LNZ308 used for activation screens were transduced with lentiviral particles to stably express dCas9-VP64 prior to screening according to the protocol outlined above (Addgene plasmid #61425; RRID:Addgene_61425).

For genome-wide activation screens, the inventors transduced a total of 450*10^6 (LN229) and 180*10^6 (LNZ308) dCas9-VP64 expressing cells with a predetermined appropriate volume of lentiviral-packaged Calabrese library to achieve a maximum 30% transduction efficiency maintaining a 500x library coverage. They performed transductions in technical duplicates. 24 h after transduction, the inventors selected cells with a predetermined concentration of puromycin for a total of five days, and transduction efficacy was evaluated using an in-line assay. Starting on day 7 of the screen, for each duplicate they split 30*10^6 cells to drug or corresponding DMSO arms, thereby maintaining a coverage of 500x. The inventors regularly split all screen arms under continuous drug or vehicle treatment for a total of 14 days. In DMSO control arms, a minimum library coverage of 500x was kept at all times. Due to cytotoxic drug concentrations, drug arms fell under the 500x coverage early during the screen, and all surviving cells were kept throughout the entire screen. For DMSO control arms 50*10^6 cells, for treatment conditions all remaining cells were collected, pelleted and stored at -80°C for DNA extraction.

### CRISPR Cas9 screen analysis

The inventors extracted the DNA from screen pellets using the QIAmp DNA Blood Kit (Qiagen, Venlo, NL) according to manufacturer's protocol. They subjected the resulting DNA to next generation sequencing using a known protocol. Amounts of DNA subjected to sequencing were estimated to provide a 500x library coverage, assuming 6.6 pg of genomic DNA per eukaryotic cell. Additionally, sequencing data from the corresponding plasmid pools of Brunello and Calabrese libraries used to generate the lentiviral particles used in this study were provided. After next generation sequencing (NGS), quality control assessments were done using FastQC (v0.11.9). Reads were mapped to the corresponding sgRNA libraries and counted using PoolQ (3.4.3). PoolQ processed in average a total of 1.6 million reads, with an average mapping rate of 75.5%. Each sgRNA instance was counted and a tab delimited count file was generated. The inventors used a custom script for file and table formatting. Log2 fold changes from both drug and DMSO arm replicates compared to the plasmid reference and corresponding reads from CRISPR/Cas9 knockout studies were corrected for gene independent effects using crisprcleanR (v3.0.1). Then, corrected reads counts were further analyzed using the MAGeCK MLE algorithm with the MAGeCK software (0.5.9.5) to identify screen hits. MAGeCK MLE utilizes a maximum-likelihood estimation (MLE) for robust identification of CRISPR-screen hits and assigns beta scores and corresponding FDR statistics to assess gene depletion or enrichment in the screens. Screens were compared to either the corresponding DMSO controls or the plasmid reference as indicated by the design matrices used for MAGeCK MLE. Screen visualization was generated with MAGeCKFlute (3.16). Genes further considered all presented an FDR value below 10% when compared with plasmid or DMSO.

### Functionally-instructed design of compound library for drug screens

The inventors leveraged an acute cytotoxicity assay workflow to evaluate potential interaction partners for AZD6738 which were derived from transcriptomic, proteomic and genome-wide CRISPR/Cas9 genetic dependency analyses. In a 1×1/IC50×IC50 set-up, they treated LN229 and LNZ308 cells in serum free medium for 72 h. The inventors used the original Bliss Independence Criterion to evaluate synergism potential. For this, a predicted value for additivity, derived from the product of the two monotherapy settings, was compared to the actual measurement of the combined treatment of the two drugs. Lower values than predicted point towards synergy, higher values towards antagonism; see Yan et al. (2010), A formal model for analyzing drug combination effects and its application in TNF-α-induced NFκB pathway. BMC Systems Biology 4: 50. Subsequently, the inventors subjected LN229, LNZ308, GS-2 and GS-9 cells to 4x4 synergy map assays again in an acute cytotoxicity assay workflow. The inventors used the R package synergyfinder with zero interaction potential (ZIP) synergy scores calculated from 4x4 concentration matrices for each tested combination and the average across the plate; see He et al. (2018); Methods for High-throughput Drug Combination Screening and Synergy Scoring. In Cancer Systems Biology: Methods and Protocols, von Stechow (ed) pp 351-398. New York, NY: Springer New York. Averages were used to produce an overview heatmap of the drug screen and evaluate the best combination approach.

### Zebrafish experiments

The inventors used zebrafish wildtype (wt) TE embryos younger than 5 days post-fertilization (dpf). Zebrafish lines were kept according to standard protocols and handled in accordance with European Union animal protection directive 2010/63/EU and approved by the local government. The inventors stably transduced cells with pLJM1-EGFP (Addgene plasmid #19319; RRID:Addgene_19319). Labelled cells were suspended in PBS at 2*10⁵ cells/µL and approx. 1 nL cell suspension was injected into the midbrain region of 24 hpf embryos. The inventors incubated the embryos at 28°C for 1 h and afterwards divided them into treatment groups. Only successfully transplanted embryos were used for further experiments. Then, the inventors applied the treatments as indicated in the respective figures and incubated that for 48 h at 35°C. For evaluation of tumor size, they imaged embryos on a Nikon Stereomicroscope (SMZ18) with the NIS Element software. The inventors assessed the tumor surface areas using Imaris (version 9.2.0).

### Statistical analysis

For statistical analyses the inventors used Gehan-Breslow-Wilcoxon test, multiple unpaired t tests, Mann-Whitney test, one-way ANOVA, Fisher's exact test as suitable and indicated in the respective figure legends. All replicates are derived from distinct samples and Gaussian distribution of all samples was assumed. The inventors assumed significance when adjusted p-values were below 0.05, shown are mean ± SD or median values and all normalizations are indicated in the figure legends. For animal experiments, they performed a biostatistical assessment. For sample size planning we aimed at a power of 80%, assuming normal distribution and standard deviation based on previous experiments for the time until onset of neurological symptoms.

### 3. Results

### Anti-glioma activity of ATR inhibition in experimental glioma in vivo, in vitro and ex vivo

Monotherapy with the ATR-inhibitor AZD6738 led to a prolongation of survival in the syngeneic SMA560/VM/Dk glioma model *in vivo* compared to vehicle (median survival AZD6738: 21 days, vehicle control: 14 days, Figure 1 a). Furthermore, the ATR inhibitors AZD6378 and Berzosertib led to significant anti-glioma activity *in vitro* in human and murine cell lines (Figure 1 b). The inventors investigated ATRi in patient-derived microtumors (PDMs) and six primary cultures (TUE-PC1-6) and observed a dose-dependent reduction of cell viability upon AZD6738 treatment (Figure 1 c, d). AZD6738 treatment led to an increased induction of apoptosis compared to vehicle-treated cells (in LN229 and LNZ308: 2-fold more, in SMA560 3-fold more, in GL261 2.5-fold more apoptotic cells). Furthermore, AZD6738 treatment led to cell cycle alterations, particularly an accumulation in S phase in human LN229 and murine GL261 cells and an accumulation of LNZ308 cells in G2-M phase. Taken together, the inventors observed a significant anti-glioma activity of ATRi in several glioma cell lines, six primary cultures and two glioblastoma PDM.

### Transcriptomic and proteomic analyses of AZD6738-treated cells reveal overlapping and distinctly regulated pathways in LN229 and LNZ308 cells

To further understand the molecular mechanisms of this anti-glioma activity, the inventors continued with transcriptomic and proteomic analyses using RNA-sequencing (RNAseq) and DigiWest protein profiling before and after AZD6738 treatment. Here, they focused on LN229 and LNZ308 cells due to their diverging response in cell cycle regulation to ATRi as well as their differing genetic profiles - LN229 carry mutated TP53 and a CDKN2A deletion, LNZ308 cells lack TP53 and PTEN - that recapitulate hallmark genetic lesions of glioblastoma. Differences in gene expression profiles were primarily governed by cell line identity rather than treatment conditions as evidenced by principal component analysis (PCA). After 72 h of treatment, the inventors detected 1048 differentially expressed genes in LN229 cells and 2401 differentially expressed genes in LNZ308 cells compared to respective vehicle treatment conditions. 341 genes were upregulated in both LN229 and LNZ308 treated cells (Fisher's exact test p=2.2*10⁻¹⁶), while 22 overlapped for the downregulated genes (Fisher's exact test p=1.697*10⁻⁷; Figure 2 a). Subsequently, Kyoto encyclopedia of Genes and Genomes (KEGG) pathway analyses revealed NF-κB, cytokine-cytokine receptor interaction, IL17 signaling pathway, Type I diabetes mellitus, MAPK signaling pathway and transcriptional misregulation in cancer as most altered pathways (Figure 2 a). Downregulated genes were significantly enriched for the Rap1 signaling pathway.

Next, the inventors investigated distinctly regulated genes across the two cell lines by leveraging the likelihood ratio test (LRT) and analyzed these hits again for KEGG pathway affiliation. The inventors detected five upregulated and one downregulated pathway in LN229, and thirteen upregulated pathways in LNZ308 of which the top six are displayed (Figure 2 b). The p53 signaling pathway was significantly upregulated in LN229 cells, but not in LNZ308 cells (Figure 2 b), being in line with the p53 status of these cells. Of note, PI3K-Akt signaling was regulated in an opposing manner in these glioma cell lines upon ATRi, with pathway activation in LNZ308 and inhibition in LN229 cells (Figure 2 b).

Based on these transcriptomic data, the inventors designed a DigiWest protein profiling analysis. The selected antibody panel covered markers for cell-cycle regulation, apoptosis, NF-κB, p53 signaling and DNA damage response.

Across both cell lines, the inventors detected a significant reduction of markers for ATR target engagement such as pATR, Chk2, and Ku80. There was a significant upregulation of NF-κB, in line with the transcriptomic data. Cleaved PARP was upregulated in both cell lines confirming the detected apoptotic response towards AZD6738 treatment (Figure 2). Upregulation of pH2A.X in both cell lines, indicate an increase in DNA damage signature (Figure 2 c). When looking at markers that were differentially regulated (Figure 2 d), LNZ308 cells display an accumulation of p16 and reduced pCDK2 (Thr160) protein levels. This signature argues for a G1 arrest, and the flow cytometry analyses show an accumulation of cells in G2. In LN229 the cell cycle regulator Survivin (BIRC5) is upregulated, CDC25C and pHistH3 (Ser10) are downregulated. Hence, for both cell lines altered regulation of the cell cycle was detected upon AZD6738 treatment, yet with differing underlying signal transduction pathways. Furthermore, in line with transcriptomic data, LN229 cells show an upregulation of p53, p21 and Bax and a downregulation of pAkt.

Taken together, the inventors detected p53-specific effects of AZD6738 treatment on transcriptomic and proteomic levels, in particular regarding cell cycle and apoptosis regulation.

### Discovery of potential modifiers of response to ATRi therapy using genome-wide CRISPR/Cas9 knockout and activation screens

Next, the inventors aimed at identifying modulators of response to ATRi in glioma cells that could further enhance the anti-glioma efficacy of ATRi. To this end, the inventors performed CRISPR/Cas9 functional genomic screens using genome-wide knockout (Brunello (Doenchi et al. (2016), Optimized sgRNA design to maximize activity and minimize off-target effects of CRISPR-Cas9. Nature Biotechnology 34: 184-191) and activation sgRNA libraries (Calabrese (Sanson et al. (2018); Optimized libraries for CRISPR-Cas9 genetic screens with multiple modalities. Nature Communications 9: 5416). In order to prioritize potentially synthetic lethal hits in knockout screens, the inventors determined prior to screening those ATRi concentrations resulting in a cytostatic effect over the course of two weeks in both glioma cell lines (LN229 and LNZ308). Accordingly, to prioritize potential resistance mechanisms in activation screens, the inventors determined prior to screening those ATRi concentrations resulting in a cytotoxic effect over the course of two weeks in both glioma cell lines (LN229 and LNZ308).

The inventors observed depletion of known pan-essential genes in both knockout screens, indicating good screen performance. They defined potential synthetic lethal genetic vulnerabilities as depleted hits in the treatment condition while unchanged in the DMSO condition. Based on this, the inventors compiled a list of treatment-related genetic vulnerabilities for each screen. Hits in DNA damage repair associated genes such as FANCA and BRCA2 (Figure 3 a, b), further endorsed the validity of the screens. When comparing the hits of both cell lines, overlapping and distinct hits were determined.

The inventors defined potential genetic resistance mechanisms as enriched genes in the treatment condition while being unchanged in the DMSO condition. Interestingly, the most prominent hits in both cell lines were ABCB1, ABCG2 and RRM1 (Figure 3 c, d). The ATP binding cassette (ABC) transporters have been reported to confer resistance to chemotherapy in several cancer entities. The finding of RRM1 as treatment-related enrichment in the activation setting complements the finding of the knockout screens where RRM1 and RRM2 knockout led to depletion under treatment (Figure 3 a, b).

### Functionally-instructed drug screen reveals cisplatin, fludarabine phosphate and hydroxyurea as most promising combination candidates for ATRi

The inventors compared all identified hits with the drug gene interaction database (DGIdb). Upon further considerations regarding druggability, they also investigated vulnerabilities that were not strictly within their borders, e.g., TOP2A, DDB1 (Figure 3 a). To this end, the inventors created a drug library of 28 compounds for further validation of functionally-instructed targets as potential combination partners for ATRi in experimental glioma (Figure 4a). To validate the hypothesized synthetic lethal interactions, they used cytotoxicity assays running for 72 h. In the pre-test screen, the inventors determined Bliss synergy scores in LN229 and LNZ308 cells for all 28 compounds combined with AZD6738 drug treatment. They identified tideglusib, harmine, doxorubicin, hydroxyurea, olaparib, temozolomide, vorinostat, cisplatin, etoposide and fludarabine phosphate as top scoring hits (Figure 4 b). The subsequent 4x4 synergy map analyses were performed in the two long term cell lines LN229 and LNZ308 as well as in the glioma stem-like cells GS-2 and GS-9. All pre-selected drugs displayed a synergistic signature in at least one of the cell lines (Figure 4 c). LN229 cells and GS-2 cells display a synergistic interaction of ATRi with temozolomide or olaparib, while LNZ308 and GS-9 do not. Hydroxyurea, cisplatin and fludarabine phosphate display a synergism signature across all four cell lines (Figure 4 c).

### Combination of AZD6738 with cisplatin or fludarabine phosphate show synergistic efficacy ex vivo and in vivo

The inventors then validated these novel functionally-instructed combination therapies in six different primary cultures TUE-PC1-6 to evaluate a potential beneficial combination efficacy of AZD6738 plus cisplatin, fludarabine phosphate or hydroxyurea. Cisplatin and fludarabine phosphate led to synergistic read-outs in all six primary cultures (Figure 5 a). Hydroxyurea led to synergistic readout in TUE-PC2, TUE-PC3 and TUE-PC6 but not inTUE-PC1, TUE-PC4, TUE-PC5. The inventors therefore selected the combination of ATRi and cisplatin for further validation in zebrafish embryos *in vivo.* To this end, they transplanted LN229-GFP cells into the midbrain of wildtype zebrafish embryos and treated those with control (untreated or DMSO), AZD6738, cisplatin or the combination of both monotherapies. Of note, both monotherapies only led to a modest decrease of tumor area, which was not significant as compared to the DMSO control (Figure 5 b). In contrast, the combination of AZD6738 and Cisplatin was significantly more efficacious in inhibiting tumor growth as compared to both control settings as well as the AZD6738 monotherapy alone, thereby validating the synergy readouts in primary cultures and highlighting a novel synergistic interaction of ATRi and cisplatin treatment in experimental glioma.

### 4. Conclusion

With the present invention, the inventors provide for the first time a combination of active ingredients comprising an ATR inhibitor and a further substance which, due to the observed clear synergistic effects of both components in the treatment and prophylaxis of gliomas, can be expected to lead to a significant therapeutic improvement over current treatment concepts. The inventors were able to demonstrate these effects not only *in vitro* but also *in vivo* using representative example combinations.

## Claims

1. A pharmaceutical composition for the treatment and/or prophylaxis of a glioma, said pharmaceutical composition comprising, in therapeutically/prophylactically effective amounts, a first active agent combined with at least a second active agent, wherein the combination of said first and said second active agent, when administered to a subject, results in a synergistic effect, wherein said first active agent is an ATR inhibitor and said second active agent is selected from the group consisting of: cytostatic agent, GSK-3 inhibitor, mTOR inhibitor, and PARP inhibitor.

2. The pharmaceutical composition of claim 1, wherein said cytostatic agent is selected from the group consisting of: cisplatin, hydroxyurea, fludarabine phosphate, vorinostat, and etoposide.

3. The pharmaceutical composition of claim 1 or 2, wherein said GSK-3 inhibitor is tideglusib.

4. The pharmaceutical composition of any of the preceding claims, wherein the mTOR inhibitor is everolismus.

5. The pharmaceutical composition of any of the preceding claims, wherein the PAPR inhibitor is olaparib.

6. The pharmaceutical composition of any of the preceding claims, wherein the ATR inhibitor is selected from the group consisting of: AZD6738 (ceralasertib), VE-822 (berzosertib), BAY-1895344 (elimusertib), VE-821, RP-3500 (camonsertib), ATR-IN-21, ATR-IN-20, ETP-46464, CGK733, NU6027, ATR-IN-14, ATR-IN-10, ATR-IN-19, ATR-IN-15, ATR-IN-17, antitumor agent-28, ATR-IN-13, and ATR-IN-18.

7. The pharmaceutical composition of any of the preceding claims, wherein the ATR inhibitor is AZD6738 (ceralasertib) and/or VE-822 (berzosertib) and the second active agent is cisplatin.

8. The pharmaceutical composition of any of the preceding claims, wherein the glioma is a glioblastoma.

9. The pharmaceutical composition of any of the preceding claims, wherein the synergistic effect is determinable by Bliss independence model and the Zero interaction potency (ZIP) model.

10. A first active agent combined with at least a second active agent for use in the treatment and/or prophylaxis of a glioma, wherein the combination of said first and said second active agent, when administered to a subject, results in a synergistic therapeutic and/or prophylactic effect, wherein said first active agent is an ATR inhibitor and said second active agent is selected from the group consisting of: cytostatic agent, GSK inhibitor, mTOR inhibitor, and PARP inhibitor.

11. The first active agent combined with at least a second active agent for use of claim 9, wherein the ATR inhibitor is AZD6738 (ceralasertib) and/or VE-822 (berzosertib) and the second active agent is cisplatin.

12. A method of treatment and/or prophylaxis of glioma in a subject in need, comprising the administration to said subject of the pharmaceutical composition of any of claims 1-9 or the first active agent combined with at least a second active agent for use of claims 10 or 11.

13. The method of claim 12, wherein the first and the second active agent are administered simultaneously.

14. The method of claim 12, wherein the first and the second active agent are administered separately.
